# EUROPÄISCHE PATENTSCHRIFT

(11) **EP 1 532 126 B1**
(45) Veröffentlichungstag und Bekanntmachung des Hinweises auf die Patenterteilung: **13.06.2007**
(21) Anmeldenummer: 03797217.1
(22) Anmeldetag: 30.07.2003
(51) Int. Cl.: C07D 307/08, C08G 65/20

(54) **VERFAHREN ZUR HERSTELLUNG VON TETRAHYDROFURAN**
METHOD FOR PRODUCING TETRAHYDROFURAN
PROCEDE DE PRODUCTION DE TETRAHYDROFURANNE

(30) Priorität: 20.08.2002 DE 10237954
(43) Veröffentlichungstag der Anmeldung: 25.05.2005
(73) Patentinhaber: BASF Aktiengesellschaft, 67056 Ludwigshafen (DE)
(72) Erfinder: PINKOS, Rolf, 67098 Bad Dürkheim (DE); KÄSHAMMER, Stefan, 67105 Schifferstadt (DE); MENGER, Volkmar, 67434 Neustadt (DE); HAUBNER, Martin, 69124 Eppelheim (DE); GROLL, Peter, 67125 Dannstadt-Schauernheim (DE); SCHLITTER, Stephan, 67117 Limburgerhof (DE); PFAFF, Klaus-Peter, 67159 Friedelsheim (DE)
(86) Internationale Anmeldenummer: PCT/EP2003/008403
(87) Internationale Veröffentlichungsnummer: WO 2004/026853

(56) Entgegenhaltungen:
- WO-A-03/099905
- DE-A- 2 916 653
- DE-C- 4 205 984
- US-A- 5 596 074
- US-A- 5 641 857
- US-B1- 6 201 137
- US-B1- 6 316 640
- DATABASE WPI Derwent Publications Ltd., London, GB; AN 1986-193392 XP002261198 & JP 61 126080 A in der Anmeldung erwähnt
- DATABASE WPI Derwent Publications Ltd., London, GB; AN 2001-183580 XP002261199 & CN 1 272 495 A (CHINA PETRO-CHEM CORP), 8. November 2000 (2000-11-08)

## Beschreibung

Die vorliegende Erfindung betrifft ein Verfahren zur kontinuierlichen Herstellung von Tetrahydrofuran durch Cyclisierung von 1,4-Butandiol.

Verfahren zur Herstellung von THF aus 1,4-Butandiol sind seit langem bekannt. In K. Weissermel, H.-J. Arpe, Industrielle Organische Chemie, VCH Verlagsgesellschaft, D-69451 Weinheim, 1994, Seite 111, ist die Umsetzung von 1,4-Butandiol zu THF durch Wasserabspaltung unter Zugabe von Phosphorsäure, Schwefelsäure oder sauren Ionenaustauschern beschrieben. Dabei wird das mit Säure versetzte 1,4-Butandiol erhitzt und in dem Maß durch 1,4-Butandiol ergänzt, wie THF/Wasser herausdestilliert.

Das Dokument US6316640 offenbart ein Verfahren zur Herstellung von THF in Gegenwart von γ Al₂O₃ Katalysator aus einer Reaktionsmischung, die außer 1 ,4-Butandiol zusätzlich weitere Nebenkomponenten wie zum Beispiel 2-(4-Hydroxybutoxy)-tetrahydrofuran enthält.

Aus JP-A 61-126 080 ist die mit Heteropolysäuren katalysierte Cyclisierung von 1,4-Butandiol zu THF bekannt. In Beispiel 4 wird zudem die Umsetzung von 1,4-Butandioldiacetat zu THF unter Wasserzusatz beschrieben. Die Umsätze zu THF liegen in allen Ausführungsbeispielen unter 100 %, so dass immer noch Ausgangsverbindung gegen Ende der Reaktion vorhanden ist. Dies ist für die großtechnische Umsetzung nachteilig, da das nicht umgesetzte Edukt entweder verworfen oder kostenaufwendig rückgeführt werden muss. Den Ausführungsbeispielen ist zu entnehmen, dass die Trocknung der Heteropolysäure vor ihrem Einsatz in der Cyclisierung erforderlich ist. Zudem ist der beschriebene Einsatz von mindestens 5 Gew.-% Heteropolysäure, bezogen auf den eingesetzten Alkohol, sehr kostenintensiv.

Nachteilig an diesem Verfahren zur Herstellung von THF ist weiterhin, dass das üblicherweise eingesetzte 1,4-Butandiol vor dem Einsatz zunächst gereinigt werden muss. Bei dieser Reinigung üblicherweise zu entfernende Nebenkomponenten des Butandiols sind 2-(4-Hydoxybutoxy)-tetrahydrofuran, ein cyclisches Acetal aus 4-Hydroxybutyraldehyd und 1,4-Butandiol (im folgenden Acetal genannt), sowie Dimere, Oligomere bzw. Polymere des Butandiols (Polytetrahydrofuran, im folgenden PolyTHF genannt), sowie Acetate bzw. Diacetate des PolyTHFs.

Diese Nebenkomponenten können die kontinuierliche Herstellung von THF beeinträchtigen. So zerfällt das Acetal unter sauren Bedingungen in 2,3-Dihydrofuran und 1,4-Butandiol, wobei das 2,3-Dihydrofuran in Gegenwart von Säuren sehr leicht polymerisiert. Diese Polymere würden sich Aufpegeln und durch Ausschleusung Verlust von Produkten und Katalysator bedeuten. In Gegenwart von Säure und Wasser wird das Acetal in 1,4-Butandiol und 4-Hydroxybutyraldehyd hydrolysiert, wobei letzterer ebenfalls polymerisieren kann.

Die Nebenkomponenten auf Basis Polytetrahydrofuran (PolyTHF) bzw. PolyTHT-Di und Monoacetate können ebenfalls zum Aufpegeln von Polymeren führen. Voraussetzung für eine technisch ausreichende Standzeit ist also eine Spaltung der PolyTHF-Derivate unter Reaktionsbedingungen.

Die Reinigung von 1,4-Butandiol erfolgt meist durch aufwendige, mehrstufige Destillation, wobei unerwünschte leicht- und/oder schwersiedende Bestandteile, einschließlich Wasser, abgetrennt werden. Anschließend wird dieses wasserfreie Rein-Butandiol zu THF umgesetzt, wobei Wasser und unerwünschte Nebenprodukte entstehen. Daher muß das als Produkt erhaltene THF nach der Umsetzung wiederum mehrstufig destillativ gereinigt werden. Es müssen also zweimal vergleichbare, aufwendige Reinigungs- und Abtrennungsschritte durchgeführt werden.

Die Aufgabe der vorliegenden Erfindung ist es daher, ein Verfahren zur Herstellung von THF in Gegenwart von Heteropolysäuren bei praktisch vollständigem Umsatz aus 1,4-Butandiol bereitzustellen, bei dem die eingesetzte, 1,4-Butandiol enthaltende Reaktionsmischung nicht vorgereinigt werden muß, und die Bildung nennenswerter Mengen von Nebenprodukten vermieden wird. Zudem sollte der Heteropolysäure-Katalysator weder vorgetrocknet noch in großen Mengen eingesetzt werden müssen.

Die Lösung dieser Aufgabe geht aus von einem Verfahren zur Herstellung von THF durch Umsetzung einer 1,4-Butandiol enthaltenden Reaktionsmischung an einem nicht vorgetrockneten Heteropolysäure-Katalysator.

Das erfindungsgemäße Verfahren hat den Vorteil, dass die eingesetzten 1,4-Butandiol enthaltenden Reaktionsmischungen vor der Umsetzung zu THF nicht vorgereinigt werden müssen. 1,4-Butandiol wird in Gegenwart von Acetal und/oder PolyTHF und/oder dessen Mono- oder Diester cyclisiert, ohne dass nennenswerte Mengen Nebenprodukte entstehen. Damit entfällt die aufwendige Vorreinigung ebenso wie die Vortrocknung des Katalysators und die Verwendung großer Katalysatormengen, so dass erhebliche Kosten gespart werden und ein wirtschaftliches Verfahren zur Verfügung gestellt werden kann.

Die bei der Umsetzung eingesetztes 1,4-Butandiol enthaltenden Reaktionsmischungen können durch bekannte Verfahren erhalten werden.

So kann beispielsweise eine 1,4-Butandiol enthaltende Reaktionsmischung eingesetzt werden, die nach dem Reppe-Verfahren aus Acetylen und Formaldehyd und anschließende Hydrierung des entstandenen 1,4-Butindiols gewonnen wird, oder durch Acetoxylierung bzw. Chlorierung von Butadien. Die nach dem Reppe-Verfahren gewonnen 1,4-Butandiol enthaltenden Reaktionsmischungen enthalten in der Regel 50 bis 3000 ppm Acetal.

Es kann auch der Hydrieraustrag der Hydrierung einer Verbindung ausgewählt aus Maleinsäure, Maleinsäuremonoestern, Maleinsäurediestern, Maleinsäureanhydrid und einem bei deren Hydrierung entstehenden Zwischenprodukt als 1,4-Butandiol enthaltende Reaktionsmischung eingesetzt werden. Solche Zwischenprodukte sind beispielsweise Bernsteinsäureanhydrid, γ-Butyrolacton, Bernsteinsäure oder Bernsteinsäurediester. Bevorzugt wird der Hydrieraustrag der Hydrierung von Maleinsäurediestern als Maleinsäure, bei der Umsetzung zu THF eingesetzt.

Die Hydrierung kann in bekannter Weise in der Gas- oder Flüssigphase durchgeführt werden. Beispielsweise kann Maleinsäuredimethylester durch Hydrierung an einem Katalysator, z.B. Kupferchromit, unter erhöhtem Druck und erhöhter Temperatur in der Gasphase umgesetzt werden. Der gewonnene Hydrieraustrag, der als Zulauf in der erfindungsgemäßen Umsetzung eingesetzt wird, enthält im allgemeinen 5-85 Gew.-% Butandiol und 15 - 95 Gew.-% Alkohol, bevorzugt 10 bis 70 Gew.-% Butandiol und 15 bis 70 Gew.-% Alkohol, besonders bevorzugt 15 bis 60 Gew.-% Butandiol und 15 bis 50 Gew.-% Alkohol. Daneben können Produkte wie γ-Butyrolacton oder Bernsteinsäurediester im Bereich bis z.B. 30 Gew.-%, enthalten sein. Die Gehalte an γ-Butyrolacton oder Bernsteinsäurediester sind im allgemeinen für das Verfahren unkritisch. Weiterhin kann Wasser in einem Gehalt von im allgemeinen unter 5 Gew.-%, bevorzugt unter 2 Gew.-%, besonders bevorzugt unter 1 Gew.-% vorhanden sein, sowie geringe Mengen weiterer Verbindungen. Es ist möglich, daß im Hydrieraustrag bereits THF vorliegt, wobei der THF-Gehalt für das Verfahren nicht kritisch ist und z.B. zwischen 10 und 30 Gew.-% liegen kann.

Anstelle des gesamten Hydrieraustrags kann auch nur ein Teilstrom des Hydrieraustrags der Umsetzung zu THF zugeführt werden. Das Reaktionsprodukt der Umsetzung zu THF kann denselben Aufarbeitungskolonnen zugeführt werden wie der nicht weiter umgesetzte Teilstrom des Hydrieraustrags, da beide ähnliche Verunreinigungen und Nebenprodukte aufweisen. So brauchen nicht für vergleichbare Trennaufgaben unterschiedliche Apparaturen betrieben zu werden.

Weiterhin kann als 1,4-Butandiol enthaltende Reaktionsmischung, die neben Acetal auch PolyTHF und/oder dessen Monoacetat oder Diacetat enthält, eine PolyTHF-haltige Reaktionsmischung verwendet werden. Derartige PolyTHF-haltige Reaktionsmischungen werden durch THF-Polymerisation an festen sauren Katalysatoren, wie beispielsweise bei T. Setoyama et al, Catalysis Today 73 (2002), Seite 29-37, beschrieben, hergestellt. Bei der Polymerisation fallen durch Nebenproduktströme Ausschleusungen, An- und Abfahrprozesse PolyTHF- und/oder PolyTHF-mono- und diacetatströme an, die verbrannt werden müssten, wenn man sie nicht wieder in THF und gegebenenfalls Essigsäure zurückspalten könnte. Diese Ströme können als 1,4-Butandiol enthaltenden Reaktionsmischung in dem erfindungsgemäßen Verfahren eingesetzt werden und enthalten zwischen 0 und 95 Gew.-% PolyTHF und/oder PolyTHF-mono- oder -diacetat, bezogen auf die 1,4-Butandiol enthaltende Reaktionsmischung, im allgemeinen jedoch nicht über 50 Gew.-%. Das PolyTHF bzw. dessen Mono- oder Diester weisen mittlere Molmassen zwischen 150 und 5000 auf.

Unter der Bezeichnung "mittleres Molekulargewicht" oder "mittlere Molmasse" wird in dieser Anmeldung das Zahlenmittel Mₙ des Molekulargewichts der im gebildeten Polymerisat enthaltenen Polymeren verstanden.

Die Umsetzung der 1,4-Butandiol enthaltenden Reaktionsmischung erfolgt bei 80 bis 300°C, bevorzugt 140 bis 250°C, besonders bevorzugt bei 150 bis 220°C. Sie wird in einem Druckbereich von 0,1 bis 15 bar, bevorzugt 0,5 bis 10 bar, besonders bevorzugt 0,8 bis 5 bar durchgeführt.

Als Katalysatoren werden Heteropolysäuren zur Umsetzung der 1,4-Butandiol enthaltenden Reaktionsmischung verwendet.

Heteropolysäuren, die erfindungsgemäß verwendet werden, sind anorganische Polysäuren, die im Gegensatz zu Isopolysäuren mindestens zwei verschiedene Zentralatome besitzen. Heteropolysäuren entstehen aus jeweils schwachen mehrbasigen Sauerstoffsäuren eines Metalles, wie Chrom, Molybdän, Vanadium und Wolfram sowie eines Nichtmetalles, wie Arsen, Jod, Phosphor, Selen, Silizium, Bor und Tellur als partielle gemischte Anhydride. Als Beispiele seine die Dodecawolframphosphorsäure H₃(PW₁₂O₄₀) oder die Decamolybdophosporsäure H₃(PMo₁₂O₄₀) genannt. Die Heteropolysäuren können als zweites Zentralatom auch Aktionoide oder Lanthanoide enthalten (s.Z. Chemie 17 (1977), Seiten 353 bis 357 bzw. 19 (1979), 308). Die Heteropolysäuren können allgemein durch die Formel H₈₋ₙ(YⁿM₁₉O₄₀) mit n = Wertigkeit des Elementes Y (z.B. Bor, Silizium, Zink) beschrieben werden (s. auch Heteropoly- und Isopolyoxomtalates, Berlin; Springer 1983). Für das erfindungsgemäße Verfahren sind als Katalysatoren auch die JP-A 61-126 080, auf die hier ausdrücklich Bezug genommen wird, genannten Heteropolysäuren. Besonders gut für das erfindungsgemäße Verfahren geeignet als Katalysatoren sind Phosphorswolframsäure und Phosphormolybdansäure wie Dodecamolybdatophosphorsäure (H₃PMo₁₂O₄₀ · nH₂O), Octadecamolybdatodiphosphorsäure (H₆P₂Mo₁₈O₆₂ · 11 H₂O), Dodecawolframatophosphorsäure H₃PW₁₂O₄₆. nH₂O) und Hexamolybdatohexawolframatophosphorsäure (H₃PMo₆W₆O₄₀ · nH₂O) .

Selbstverständlich können auch Mischungen von Heteropolysäuren eingesetzt werden. Besonders bevorzugt werden im erfindungsgemäßen Verfahren aufgrund ihrer guten Verfügbarkeit Dodecawolframatophosphorsäure und Dodecamolybdatophosphorsäure eingesetzt.

Bevorzugt werden die freien Heteropolysäuren erfindungsgemäß angewandt, es ist aber auch möglich, deren Salze, insbesondere deren Alkalimetall- und Erdalkalimetallsalze als Katalysatoren zu benutzen. Die Heteropolysäuren und deren Salze sind bekannte Verbindungen und können nach bekannten Verfahren, beispielsweise nach den Methoden vor Brauer (Herausgeber): Handbuch der Präparativen Anorganischen Chemie, Band III, s. 1774-1784, Enke, Stuttgart, 1981 oder nach den Verfahren von Top. Curr. Chem. 76, 1 (1978), hergestellt werden.

Die so hergestellten Heteropolysäuren als auch die entsprechenden Handelsprodukte enthalten 20 bis 40 Mol Wasser/Mol Heteropolysäure und werden erfindungsgemäß ungetrocknet eingesetzt. Der 1,4-Butandiol enthaltenden Reaktionsmischung wird kein Wasser zusätzlich zu dem Kristallwasser Heteropolysäure zugefügt. Erfindungsgemäß werden weniger als 1 Gew.-% Heteropolysäure, bezogen auf die 1,4-Butandiol enthaltende Reaktionsmischung, eingesetzt.

Es hat sich demnach als günstig erwiesen, den Gehalt an Heteropolysäure bei unter 1 Gew.% zu halten. Bevorzugt werden pro kg 1,4-Butandiol enthaltende Reaktionsmischung 50 - 10000 mg, besonders bevorzugt zwischen 500 und 5000 mg Heteropolysäure pro 1,4-Butandiol enthaltende Reaktionsmischung eingesetzt. Überraschenderweise ist trotz des geringen Gehalts an Heteropolysäuren die Reaktionsgeschwindigkeit hoch. Ein technisch üblicheres und besseres Maß für den mengenmäßigen Einsatz von Heteropolysäure bei einem kontinuierlichen Verfahren ist die Gesamtmenge Heteropolysäure pro umgesetztes kg Butandiol, auch als Einsatzzahl bezeichnet. Die Gesamtmenge an Heteropolysäure pro kg umgesetzten Butandiol liegt zwischen 0,1 und 200 mg, bevorzugt zwischen 0,5 und 100 mg, besonders bevorzugt zwischen 1 und 50 mg.

Erfindungsgemäß wurde erkannt, dass das erfindungsgemäße Verfahren dann besonders wirtschaftlich mit hohen Standzeiten des Katalysators verläuft, wenn die 1,4-Butandiol enthaltende Reaktionsmischung basische Stickstoffkomponenten in einer Menge von kleiner 1 ppm enthält. Die Menge enthaltenen Stickstoffkomponenten kann nach der von F. Ehrenberger in Quantitative organische Elementaranalyse, ISBN 3-527-28056 - Kapitel 37, Seite 382, beschriebenen Methode bestimmt werden.

Bei der technischen Umsetzung des erfindungsgemäßen Verfahrens, wird in aller Regel ein metallischer Reaktor verwendet. Da eine metallische Oberfläche Metallionen abgeben kann, insbesondere dann, wenn Säuren zugegen sind, wie z.B. Essigsäure die durch Spaltung von PolyTHFacetaten stammt, kommt es im Laufe der Zeit zu einer Desaktivierung der erfindungsgemäß eingesetzten Heteropolysäure durch Metallionen. Dieser Effekt kann im Übrigen auch durch aminhaltige Komponenten in den Zulaufströmen eintreten. Um nun die Aktivität der Heteropolysäure zu erhalten, kann der flüssige Inhalt des Reaktors über einen Feststoff geleitet werden, der in der Lage ist, Kationen z.B. Eisens, Nickels, Chroms, Aluminiums sowie auch Ammoniumionen aufzunehmen. Solche Feststoffe sind z.B. typische Ionentauscher in protonierter Form auf organischer Basis, die kommerziell erhältlich sind. Beispiele hierfür sind die Handelsprodukte Lewatite der Firma Bayer AG, Leverkusen, Amberliste der Firma Röhn und Haas GmbH, Darmstadt oder Nafion der Firma E.I. du Pont de Nemours. Es können selbstverständlich auch anorganische Ionentauscher wie z.B. des Handelsprodukts Deloxane der Firma Degussa AG, Hanau, Tonerden und Zeolithe in der H-Form verwendet werden.

Das nach dem erfindungsgemäßen Verfahren enthaltene THF kann für weitere Zwecke, z.B. als Lösungsmittel verwendet werden. Besonders bevorzugt ist die Verwendung des nach dem erfindungsgemäßen Verfahren erhaltenen THF's zur Herstellung von PolyTHF, dessen Mono- oder Diestern.

Besonders bevorzugt wird das erfindungsgemäß erhaltene THF vor seinem Einsatz in der Polymerisation zu PolyTHF, seinen Mono- oder Diester einer Hydrierung unterzogen.

Dabei wird das THF z.B. an Ni, Pd oder Cu enthaltenden Katalysatoren in an sich bekannter Art und Weise hydriert. Es hat sich überraschend gezeigt, dass dadurch die Polymerisation, die beispielsweise in DE 2 916 653 A1 beschrieben ist, mit längeren Katalysatorstandzeiten und niedrigeren Farbzahlen im Produkt einhergeht.

Der Umsatz des in der Reaktionsmischung vorhandenen 1,4-Butandiols zu THF beträgt im allgemeinen 99 bis 100 %. Somit entspricht der Austrag nach der Umsetzung von 1,4-Butandiol zu THF (Cyclisierungsaustrag) im wesentlichen seiner Zulaufzusammensetzung, mit dem Unterschied, daß das im Zulauf enthaltene 1,4-Butandiol zu THF und Wasser umgesetzt wurde. Der Cyclisierungsaustrag enthält im allgemeinen THF, Reaktionswasser und geringe Mengen an 2,3-Dihydrofuran.

Anhand der Zusammensetzung des Cyclisierungsaustrags wird deutlich, daß bei der Umsetzung von 1,4-Butandiol enthaltenden Reaktionsmischungen zu THF in Gegenwart von Heteropolysäuren keine nennenswerte Bildung von Nebenprodukten, wie von Ethern durch intermolekulare Reaktion, auftritt.

Der Cyclisierungsaustrag kann mit dem Fachmann bekannten Methoden destillativ aufgearbeitet werden. EP-B 0 485 484 beschreibt verschiedene Verfahren zur Gewinnung von THF aus Mischungen, die THF, einen oder mehrere niedrigsiedende Alkohole und Wasser enthalten. So kann die Gewinnung beispielsweise durch extraktive Destillation unter Zugabe einer weiteren Komponente wie 1,4-Butandiol, Wasser, Ethylenglykol und anderen erfolgen.
EP-B 0 485 484 beschreibt weiterhin ein Verfahren zur Gewinnung von THF aus den erwähnten Mischungen, das zwei aufeinanderfolgende Destillationen, wobei die erste bei niedrigerem Druck als die zweite durchgeführt wird, und eine zwischen den Destillationen durchgeführte Kondensation umfasst. Das aus der zweiten Destillation gewonnene, an Nebenprodukten angereicherte Gemisch wird mit dem Strom der ersten Destillation wiederum kondensiert, und das in der zweiten Destillation gewonnene reine THF wird abgetrennt.

Die nachfolgenden Beispiele sollen die Erfindung zusätzlich erläutern.

### Beispiele

Die Prozentangaben des Butandiols bzw. der Reaktionsausträge sind durch Gaschromatographie ermittelte GC-Flächenprozente.

Das in den Beispielen verwendete 1,4-Butandiol ist Handelsprodukt der BASF AG/Ludwigshafen und hatte folgende Zusammensetzung: 99,8 % 1,4-Butandiol, 800 ppm Acetal. Der Rest bestand überwiegend aus 2-Methylbutandiol.

### Beispiel 1

Ein 250 ml Glaskolben wurde zur Hälfte mit etwa 100 g 1,4-Butandiol gefüllt, das 800 ppm Acetal enthielt und dessen Gehalt an basischen Stickstoffkomponenten < 1 ppm war. Dazu wurden 100 mg handelsübliche nicht entwässerte Dodecawolframatophosphorsäure gegeben. Das Gemisch wurde auf 175 - 180°C aufgeheizt. Nach kurzer Zeit destillierte ein Gemisch aus THF und Wasser ab, das noch 1000 ppm 1,4-Butandiol, 450 ppm 3-Methyltetrahydrofuran und ca. 300 ppm 2,3-Dihydrofuran enthielt. In dem Maße wie Produkt abdestilliert, wurde frisches Butandiol kontinuierlich über eine Pumpe zugeführt (ca. 15 g/h). Der Austrag erfolgte nur über die Gasphase, d.h. es wurden keine Hochsieder über die Flüssigphase ausgeschleust. Nach 500 Betriebsstunden entsprach die Zusammensetzung des Reaktionsaustrags immer noch der zu Beginn der Umsetzung erhaltenen. Auch die Produktivität entsprach unverändert derjenigen der ersten Stunde. Es konnten also mit nur 100 mg Heteropolysäure 7,5 kg Butandiol in 500 Betriebsstunden umgesetzt werden, ohne dass die Aktivität der Heteropolysäure gelitten hätte, d.h. für ein kg Butandiol wurden 13,3 mg Heteropolysäure benötigt.

### Beispiel 2

Analog Beispiel 1 wurde unter den Bedingungen aus Beispiel 1 1,4-Butandiol umgesetzt. Nach 48 Stunden wurde der Reaktionszulauf auf ein Gemisch umgestellt, das aus 80 Gew.-% 1,4-Butandiol, 13 % THF und PolyTHF-di- und monoacetaten mit einem mittleren Molgewicht von 460 bestand. Bei 180°C Reaktionstemperatur wurden nach weiteren 96 Stunden neben THF und Wasser laut GC-Analyse 300 ppm 1,3-Dihydrofuran, 500 ppm 1,4-Butandiol, 900 ppm 3-Methyltetrahydrofuran sowie einige unbekannte, mengenmäßig untergeordnete Verbindungen gefunden. Die Katalysatorproduktivität war wie zu Beginn.

### Beispiel 3 Verwendung

Gemäß Beispiel 2 hergestelltes THF wurde mit 40%iger Natronlauge zweimal gewaschen und anschließend bei ca. 65°C Kopftemperatur und 1013 mbar destilliert. Das so erhaltene THF enthielt 90 ppm 2,3-Dihydrofuran. 500 g dieses THF's wurden analog Beispiel 1b) der DE 2 916 653 polymerisiert. Der anfängliche Umsatz von ca. 34 % fiel bereits nach wenigen Stunden ab. Nach 24 h wurde der Versuch abgebrochen. Der Umsatz war auf 30 % gesunken und der Polymerisationsaustrag war gelb gefärbt.

### Beispiel 4

1000 g eines gemäß Beispiel 3 durch Behandlung mit Natronlauge und anschließende Destillation gereinigten THF's aus Beispiel 3 wurde bei 10 bar, 60°C, an einem Katalysator der folgenden Zusammensetzung (oxidisch) 21,5 % NiO, 7,3 % CuO, 2,0 % Mn₃O₄, 1,2 % H₃PO₄, Rest SiO₂ hydriert, der zuvor mit Wasserstoff bei 180°C aktiviert worden war. Anschließend wurde das THF wie in Beispiel 3 polymerisiert. Der Katalysator zeigte auch nach 48 h Betriebszeit noch kein Nachlassen der Aktivität und die Reaktionsausträge waren farblos.

### Beispiel 5

Beispiel 2 wurde wiederholt, allerdings unter Zusatz von 50 ml Drahtspiralen V4A (1.4571) (Simulation eines Reaktors aus Edelstahl). Nach 700 h betrug die Katalysatoraktivität noch ca. 40 % der Ausgangsaktivität. Daraufhin wurde die Reaktionsmischung auf ca. 25°C abgekühlt und die Flüssigphase über 50 ml eines sauren Kationenaustauscher (Amberlite-IR-120) filtriert und wieder unter den ursprünglichen Reaktionsbedingungen eingesetzt. Es stellte sich die Ausgangsaktivität wieder ein.

## Patentansprüche

1. Verfahren zur kontinuierlichen Herstellung von THF durch Umsetzung einer 1,4-Butandiol enthaltenden Reaktionsmischung an einem nicht vorgetrockneten Heteropolysäurekatalysator, **dadurch gekennzeichnet, dass** die Reaktionsmischung unter 1 ppm an basischen Stickstoffkomponenten und 2-(4-Hydroxybutoxy)-tetrahydrofuran enthält.

2. Verfahren nach Anspruch 1, **dadurch gekennzeichnet, dass** die Reaktionsmischung Polytetrahydrofuran und/oder dessen Mono- oder Diacetate enthält.

3. Verfahren nach einem der Ansprüche 1 bis 2, **dadurch gekennzeichnet, dass** weniger als 1 Gew.-% Heteropolysäure, bezogen auf die Reaktionsmischung zugesetzt werden.

4. Verfahren nach einem der Ansprüche 1 bis 3, **dadurch gekennzeichnet, dass** die Umsetzung bei einer Temperatur von 80 bis 300°C durchgeführt wird.

5. Verfahren nach einem der Ansprüche 1 bis 4, **dadurch gekennzeichnet, dass** die Umsetzung in einem Druckbereich von 0,5 bis 10 bar durchgeführt wird.

6. Verfahren nach den Ansprüchen 1 bis 5, **dadurch gekennzeichnet, dass** die Reaktionsmischung bei Nachlässen der Aktivität über einen Kationenaustauscher geleitet wird.

## Claims

1. A process for the continuous preparation of THF by reaction of a 1,4-butanediol-comprising reaction mixture over a heteropolyacid catalyst which has not been predried, wherein the reaction mixture comprises less than 1 ppm of basic nitrogen components and 2-(4-hydroxybutoxy)tetrahydrofuran.

2. The process according to claim 1, wherein the reaction mixture comprises polytetrahydrofuran and/or its monoacetates or diacetates.

3. The process according to either of claims 1 and 2, wherein less than 1% by weight of heteropolyacid, based on the reaction mixture, is added.

4. The process according to any of claims 1 to 3, wherein the reaction is carried out at from 80 to 300°C.

5. The process according to any of claims 1 to 4, wherein the reaction is carried out in a pressure range from 0.5 to 10 bar.

6. The process according to any of claims 1 to 5, wherein the reaction mixture is passed over a cation exchanger after the activity decreases.

## Revendications

1. Procédé de préparation en continu de THF par réaction d'un mélange réactionnel contenant du 1,4-butanediol sur un catalyseur d'hétéropolyacide non séché au préalable, **caractérisé en ce que** le mélange réactionnel contient moins de 1 ppm de composés azotés basiques et de 2-(4-hydroxybutoxy) tétrahydrofuranne.

2. Procédé suivant la revendication 1, **caractérisé en ce que** le mélange réactionnel contient du polytétrahydrofuranne et/ou ses mono- ou diacétates.

3. Procédé suivant l'une quelconque des revendications 1 à 2, **caractérisé en ce que** l'on ajoute moins de 1% en poids d'hétéropolyacide, par rapport au mélange réactionnel.

4. Procédé suivant l'une quelconque des revendications 1 à 3, **caractérisé en ce que** la réaction est effectuée à une température de 80 à 300°C.

5. Procédé suivant l'une quelconque des revendications 1 à 4, **caractérisé en ce que** la réaction est effectuée dans une plage de pression de 0,5 à 10 bar.

6. Procédé suivant l'une quelconque des revendications 1 à 5, **caractérisé en ce que** le mélange réactionnel est envoyé sur un échangeur de cations en cas de relâchement de l'activité.
